# EUROPEAN PATENT SPECIFICATION

(11) **EP 3 476 405 B1**
(45) Date of publication and mention of the grant of the patent: **21.10.2020**
(21) Application number: 18203040.3
(22) Date of filing: 29.10.2018
(51) Int. Cl.: A61L 2/26, E05D 3/02, E05D 7/081, A47L 15/42

(54) **MACHINE FOR TREATING OBJECTS, IN PARTICULAR FOR WASHING, THERMAL DISINFECTION AND/OR STERILIZATION OF OBJECTS**
MASCHINE ZUR BEHANDLUNG VON OBJEKTEN, INSBESONDERE ZUM WASCHEN, ZUR THERMISCHEN DESINFEKTION UND/ODER STERILISIERUNG VON OBJEKTEN
MACHINE DE TRAITMENT D'OBJECTS, NOTAMMENT DE LAVAGE, DE DÉSINFECTION THERMIQUE ET/OU DE STÉRILISATION D'OBJETS

(30) Priority: 30.10.2017 IT 201700123436
(43) Date of publication of application: 01.05.2019
(73) Proprietor: Steelco S.p.A., 31039 Riese Pio X (IT)
(72) Inventor: Capovilla, Ivone, 31037 Loria (TV) (IT)
(74) Representative: Petraz, Gilberto Luigi

(56) References cited:
- EP-A1- 0 744 521
- EP-A2- 2 687 660
- US-A- 4 979 265
- US-A1- 2016 346 415
- US-A1- 2017 152 690

## Description

### FIELD OF THE INVENTION

The present invention concerns a machine for treating objects provided with at least one treatment chamber with which fluidic sealing means, for example gaskets or other, are associated.

The term "treatment" can include "in their entirety" both operations for the pre-treatment of objects, such as pre-washing, with hot or cold water and/or with detergents or other chemical products, and also actual washing operations, and drying operations. Also included in the same term "treatment" are thermal disinfection operations, for example sterilization, and decontamination by means of particularly aggressive and dangerous decontaminating substances, detergents and/or chemical agents, such as for example peracetic acid.

By way of example, the objects that can be treated in the treatment machine discussed can be instruments used in the health sector, in the laboratory, for analysis or for research, instruments used in the pharmaceutical sector, or instruments in the medical sector, surgical instruments or similar or comparable instruments, but without excluding the application of the present invention to the treatment of objects in general.

### BACKGROUND OF THE INVENTION

Treatment machines are known having a treatment chamber which is accessed through an aperture, typically at the front, for the insertion through it of the objects to be treated and provided with a closing device formed by a rotatable door, which can be a swing door, that is, with a vertical axis of rotation, or a flap door, that is, with a horizontal axis of rotation, or suchlike.

The rotatable door is hinged along one of the peripheral edges which delimit the aperture and cooperates with the peripheral edges of the aperture.

Between the rotatable door, for example a flap door, swing door or suchlike, and the aperture of the treatment chamber, the fluidic sealing means can be positioned, for example a gasket, generally made of rubber or similar elastomer material.

The gasket can be disposed along a continuous seating made on the entire external perimeter of the rotatable door or on the external perimeter of the aperture of the treatment chamber.

The gasket can be of the pneumatic type, and is associated with pneumatic means, such as a compressed air connection device, able to introduce a compressed fluid, typically air, inside the gasket, so as to define a first dilated operating condition of the gasket which, when the rotatable door is closed, guarantees the fluidic seal by cooperating with the peripheral edges of the aperture. Once the compressed air is released, the dilated operating condition is reversible in a substantially elastic manner, moving into a second inactive condition, when it becomes necessary to open the door.

However, the gasket, as we said, could also be a normal gasket made of rubber or other elastomer positioned between the rotatable door and the perimeter of the aperture of the treatment chamber.

A common disadvantage of such treatment machines, which use these fluidic sealing means, of pneumatic type or not, is that the fluidic sealing means can undesirably interfere with the corner of the rotatable door nearest the axis of rotation or hinging of the rotatable door, in particular during the operation of opening the rotatable door.

This interference of the corner of the rotatable door with the fluidic sealing means can lead to an excessive compression or crushing of the fluidic sealing means.

This disadvantage, naturally, can lead to a rapid deterioration of the fluidic sealing means and therefore to the need to carry out a frequent replacement thereof.

This disadvantage can also arise with the use of the pneumatic gaskets mentioned above, while providing for their deflation and then elastic return in the inactive position to allow the rotatable door to be opened.

In any case, the need to provide the elastic return of the pneumatic gasket, by deflating it, naturally determines the need for preliminary operations before a normal opening of the rotatable door can be made.

In general, therefore, with normal rotatable doors, the integrity and efficiency of the fluidic sealing means, whether pneumatic or not, is not always guaranteed in an optimal manner.

Some examples of known rotatable doors, or devices to rotate rotatable doors, which however do not solve the above problems of rotatable doors associated with fluidic sealing means and used in machines for treating objects, are described and shown in US-A documents -2016/346415, EP-A-0744521, EP-A-2687660, US-A-2017/152690 and US 4979265 A.

Other limitations and disadvantages of conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

There is therefore a need to obtain a treatment machine, in particular for washing, thermal disinfection and/or sterilization of objects, which can overcome at least one of the disadvantages of the state of the art.

One purpose of the present invention is therefore to provide a treatment machine, in particular for washing, thermal disinfection and/or sterilization of objects, provided with a device for hinging the rotatable door to the access aperture of the treatment chamber which guarantees the integrity of the fluidic sealing means disposed between the access aperture of the treatment chamber and the rotatable door, in each movement step of the rotatable door, therefore both when closing and opening.

Therefore, one purpose of the invention is to provide a treatment machine in which the integrity of the fluidic sealing means is preserved and in which, therefore, the fluidic sealing means do not run the risk of being unwantedly and excessively compressed by the movement of the rotatable door, especially in proximity to the corner of the rotatable door where the hinging or rotation axis of the rotatable door is located.

Another purpose of the invention is also to provide a treatment machine, in particular for washing, thermal disinfection and/or sterilization of objects, which allows to use any type of gasket or other fluidic sealing means, hence of a pneumatic type or not, guaranteeing optimal functioning efficiency thereof, even for prolonged use over time.

Another purpose of the present invention is also to provide a treatment machine, in particular for washing, thermal disinfection and/or sterilization of objects, which, when using fluidic sealing means of a pneumatic type, allows at least to limit, if not even to exclude, the elastic return, or deflation, of the fluidic sealing means.

Another purpose of the present disclosure is to provide a hinging device for a machine for treating objects, in particular for washing, thermal disinfection and/or sterilization of objects, which allows to optimize the use of the fluidic sealing means and maintain their integrity during all the steps of opening or closing the rotatable door, for example a swing door, a flap door or suchlike.

The Applicant has devised, tested and embodied the present invention to overcome the shortcomings of the state of the art and to obtain these and other purposes and advantages.

### SUMMARY OF THE INVENTION

The present invention is set forth and characterized in the independent claim, while the dependent claims describe other characteristics of the invention or variants to the main inventive idea.

In accordance with the above purposes, a machine for treating objects, in particular for washing, thermal disinfection and/or sterilization of objects, according to the present invention, comprises a treatment chamber provided with an access aperture positioned on a corresponding lying plane; a rotatable door configured to rotate with respect to a corresponding hinging axis in order to open and close the access aperture; and fluidic sealing means positioned between the access aperture and the rotatable door.

According to the invention, the rotatable door of the treatment machine is associated with the access aperture by a hinging device provided with a movement unit to move the hinging axis of the rotatable door with respect to the lying plane, so that the hinging axis can be distanced from the lying plane following an opening rotation of the rotatable door and can move closer to the lying plane following a closing rotation of the rotatable door.

In the present invention, the movement unit is an actuator to convert the rotary motion of the rotatable door into a two-directional linear translation motion of the hinging axis, so that the hinging axis is at a minimum distance from the lying plane when the rotatable door is closed, and at a maximum distance from the lying plane when the rotatable door is completely open.

Advantageously, by means of this combined rotation and translation movement, it is possible to distance the corner of the rotatable door from the fluidic sealing means, above all during the operations of opening the rotatable door, thus maintaining the integrity of the fluidic sealing means and preventing undesired squeezing or compression. Moreover, if the fluidic sealing means are a gasket of the pneumatic type, by means of the movement unit it is possible to proceed with opening the rotatable door without providing for operations to deflate the gasket.

The present machine also comprises at least one adjustment device configured to adjust the position of the movement unit with respect to the lying plane and the fluidic sealing means.

The adjustment device comprises attachment elements inserted into suitable slits made on gears of the movement unit, namely on the second pair of gears associated with a wall of the treatment chamber.

The attachment elements and the slits can be aligned in the direction of two-directional translation of the hinging axis.

In the adjustment device at least one pair of slits can be aligned in the direction of two-directional translation of the hinging axis.

The movement unit comprises at least a first pair of gears associated with the rotatable door and able to engage with a second pair of gears associated with a wall of the treatment chamber.

In some embodiments, the gears can comprise at least a first pair of toothed wheels positioned on the hinging axis and able to engage in corresponding racks associated with the wall of the treatment chamber.

The toothed wheels can each be associated with a lateral wall of the rotatable door and in proximity to a side of the rotatable door, the lower side can be positioned in proximity to the hinging axis of the rotatable door.

Each of the toothed wheels can be able to protrude from a guide groove made on a plate associated with the wall of the treatment chamber.

The racks can be associated, by means of the attachment elements inserted in the slits, with the plate associated with the wall of the treatment chamber, so as to allow an adjustment of the position of each rack with respect to the plate associated with the wall of the treatment chamber.

The present disclosure (not falling under the scope of the appended claims) also concerns a hinging device for a rotatable door of a treatment machine for treating objects, in particular for washing, thermal disinfection and/or sterilization of objects as defined heretofore, provided with a movement unit of the hinging axis of the rotatable door with respect to the lying plane in which the access aperture is positioned that is opened and closed by the rotatable door, so that the hinging axis can be distanced from the lying plane following an opening rotation of the rotatable door and can move closer to the lying plane following a closing rotation of the rotatable door.

The present disclosure (not falling under the scope of the appended claims) also concerns a closing unit of a treatment machine for treating objects, in particular for washing, thermal disinfection and/or sterilization of objects comprising a rotatable door and a hinging device as defined heretofore.

These and other aspects, characteristics and advantages of the present disclosure will be better understood with reference to the following description, drawings and attached claims. The drawings, which are integrated and form part of the present description, show some forms of embodiment of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a schematic three-dimensional view of a machine for treating objects, in particular for washing, thermal disinfection and/or sterilization of objects according to the invention;
- fig. 2 is an enlarged three-dimensional view of a hinging device of the rotatable door;
- figs. 3 to 5 are enlarged views, in lateral elevation and partly in section, of three different positions of the rotatable door.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We will now refer in detail to the various embodiments of the present invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

With reference to the attached drawings, a treatment machine 10 for objects, for example washing, heat-disinfection, sterilization or other, comprises a treatment chamber 11 provided with an access aperture 12, which can be opened or closed by a rotatable door 13, for example a flap door, or a swing door, depending on the disposition of the hinging axis.

The rotatable door 13 can be delimited by an upper side 16, a lower side 16', and lateral sides 16".

The sides 16, 16' and the lateral sides 16" can be formed by suitable shaped profiles or bars, for example made of metal material.

In particular, the rotatable door 13 can be rotated opening or closing around a respective hinging axis A.

Since in the example shown the rotatable door 13 is the flap type, the hinging axis A is substantially horizontal and, in this case, is positioned in correspondence with the lower side 16' of the rotatable door 13.

Alternatively, the rotatable flap door 13 could rotate around a hinging axis positioned in correspondence with the upper side 16.

If the rotatable door 13 was a swing door, on the contrary, the hinging axis A would be disposed substantially vertical, so as to rotate around one of the lateral sides 16".

The access aperture 12 of the treatment chamber 11 is positioned in a corresponding lying plane P.

The access aperture 12 comprises on the edge a housing seating 14, or labyrinth, in which fluidic sealing means 15 are inserted, for example at least one gasket or suchlike.

Naturally, it would be possible to provide that the housing seating 14, which is annular, is also made, or alternatively made, along the sides 16, 16' of the rotatable door 13 facing toward the lying plane P.

The fluidic sealing means 15 can be one or more traditional gaskets made of elastomer material, for example O-rings or suchlike, or they can be gaskets of a pneumatic type, or other.

If the fluidic sealing means 15 are the pneumatic type, it can be provided that they are connected to their own pneumatic means, such as for example compressed air pneumatic means, suitable to introduce air or other suitable fluid under pressure into the cavities of the fluidic sealing means 15.

The selective introduction of pressurized fluids into the fluidic sealing means 15 by the pneumatic means, which can be activated once the rotatable door 13 is closed, determines a dilated operating condition thereof, so as to cooperate in compression with the sides 16, 16' of the rotatable door 13, thus guaranteeing the fluidic seal.

When it is necessary to open the rotatable door 13, for example at the end of a determinate treatment cycle, or in the event of extraordinary intervention, the pneumatic means can be configured to release the pressure of the fluid from the fluidic sealing means 15, so that the fluidic sealing means 15 are taken into a second inactive condition, not dilated.

The second inactive condition is obtained in a substantially reversible manner also thanks to the elastomer or similar material of which the fluidic sealing means 15 are made.

As we said above, the dilation operation of the fluidic sealing means 15 naturally entails a series of operations on the treatment machine 10 to be carried out before opening the rotatable door 13 and, in any case, even with these preliminary operations, the integrity of the fluidic sealing means 15 is not always guaranteed, in particular in a zone close to a corner 17 of the lower side 16' of the rotatable door 13.

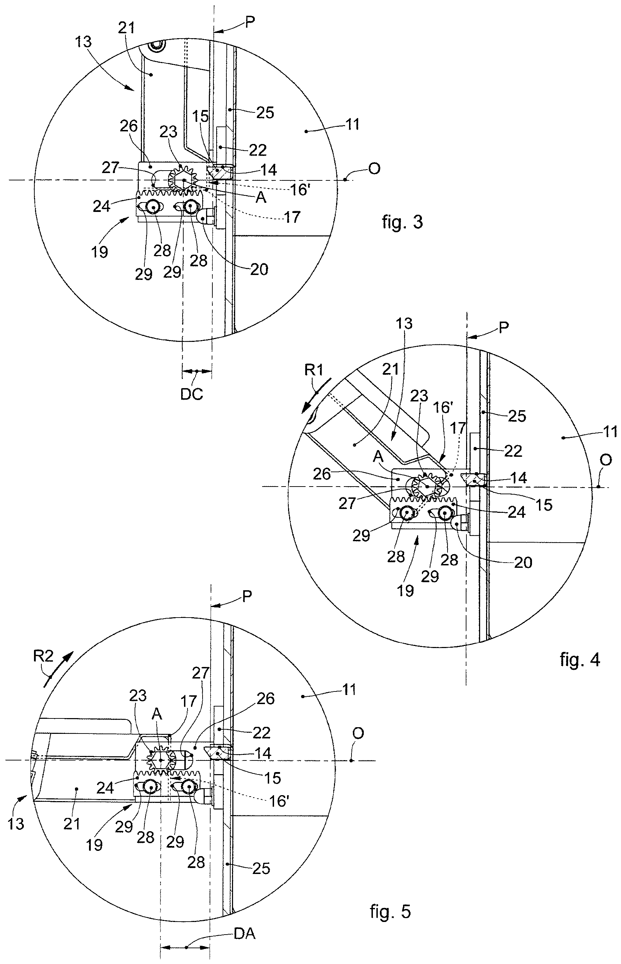

In particular, the corner 17 is the corner of the lower side 16' closest to the lying plane P in the closed position of the rotatable door 13, as shown in fig. 3.

The rotatable door 13 comprises suitable drive means 18, to allow it to pass from an open position to a closed position and vice versa.

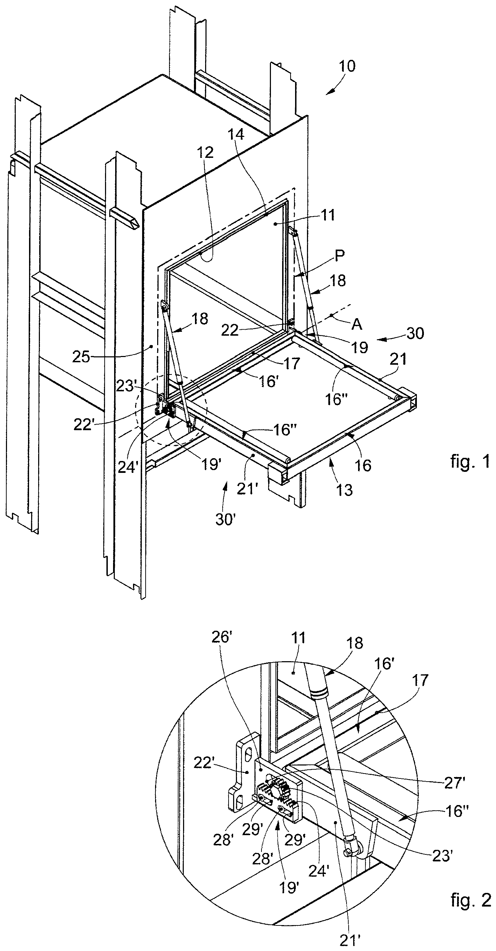

A completely open position of the rotatable door 13 is shown for example in fig. 1, fig. 2 and fig. 5.

A completely closed position of the rotatable door 13 is shown for example in fig. 3.

The rotatable door 13 is associated with the access aperture 12 by means of a hinging device 19, 19' comprising the hinging axis A of the rotatable door 13 and configured to move the hinging axis A of the rotatable door 13 away from or toward the lying plane P.

In essence, the hinging device 19, 19' is configured to take the hinging axis A closer to the lying plane P of the access aperture 12, when the rotatable door 13 is closing, while it is configured to move the axis of hinging A away from the lying plane P, when the rotatable door 13 is opening.

Substantially therefore, the rotatable door 13 and the hinging device 19, 19' constitute a closing unit 30, 30' of the treatment machine 10, in particular of the corresponding treatment chamber 11.

Fig. 3 shows the rotatable door 13 in the closed position, therefore the hinging axis A is at the minimum distance DC from the lying plane P.

Fig. 5 shows the rotatable door 13 in a completely open position, therefore the hinging axis A is at the maximum distance DA from the lying plane P.

This distancing of the hinging axis A during the opening of the rotatable door 13 advantageously allows to prevent the interference of the rotatable door 13 with the fluidic sealing means 15, in particular the interference of the corner 17 of the rotatable door 13 closest to the fluidic sealing means 15.

The hinging device 19, 19' is provided on both lateral sides 16" of the rotatable door 13, in particular on lateral walls 21, 21' of the rotatable door 13.

The hinging device 19, 19' comprises a first gear, for example a toothed wheel 23, 23', cooperating with a second gear, for example a rack 24, 24'.

The toothed wheels 23, 23' of the rotatable door 13 are positioned along the hinging axis A.

The toothed wheels 23, 23' engage in corresponding racks 24, 24', preferably rectilinear, therefore able to allow a linear two-way roto-translation movement of the toothed wheels 23 and 23', for example in direction O.

The toothed wheels 23, 23' cooperating with the racks 24, 24' therefore represent an example of a unit for moving the hinging axis A with respect to the lying plane P.

The movement unit 23, 23', 24, 24', therefore, is substantially configured as an actuator for converting a rotary motion of the rotatable door 13 into a translational motion of the hinging axis A.

By means of the movement unit 23, 23', 24, 24', moreover, it is advantageously possible to adjust the initial position of the hinging axis A, as a function, for example, of the type of fluidic sealing means 15, for example of their thickness, or the fact they are pneumatic or not, or another type.

The conversion actuator could also be different from what is shown here, and selected for example from a group comprising: screw actuators, such as a screw jack, ball screw and roller screw actuators, or wheel and axle, for example a drum, gear, pulley or shaft, actuators, such as a lifting cable, a winch, a rack and a pinion assembly, a chain transmission, a strip or belt transmission, rigid chain actuators, and rigid strip or belt actuators, or other.

In the example shown, in particular, the movement unit is a roto-translation unit, since it is provided, for example, with toothed wheels 23, 23' and racks 24, 24' reciprocally cooperating.

The racks 24, 24' are attached to a plate 26, 26' by means of suitable attachment elements 28, 28'.

The plate 26, 26' is positioned in proximity to the lateral walls 21, 21' of the rotatable door 13 and comprises a guide groove 27, 27' where a portion, not visible in the drawings, can slide so as to connect the toothed wheels 23, 23' with the lateral walls 21, 21' of the rotatable door 13.

The groove 27, 27' of the plate 26, 26' substantially allows the toothed wheels 23, 23' and therefore the hinging axis A of the rotatable door 13 to perform its travel away from or toward the lying plane P.

The rack 24, 24' is positioned outside the plate 26, 26' and is attached thereto by the attachment elements 28, 28', for example pins, bolts or suchlike.

The position of the rack 24, 24' with respect to the plate 26, 26' is adjustable, thanks to the fact that the attachment elements 28, 28' pass through suitable slits 29, 29' made in the rack 24, 24'.

The slits 29, 29' and the corresponding attachment elements 28, 28' therefore represent an effective example of an adjustment device configured to adjust the position of the movement unit 23, 23', 24, 24' with respect to the lying plane P and to the fluidic sealing means 15.

The attachment elements 28, 28' are inserted in slits 29, 29' made on a pair of gears 24, 24', for example the racks 24, 24', of the movement unit 23, 23', 24, 24'.

If the movement unit comprises the racks 24, 24' and the toothed wheels 23, 23', by means of the slits 29, 29', it is possible to adjust the position of each rack 24, 24' with respect to the lying plane P and to the fluidic sealing means 15.

For example, it can be assumed that the greater the bulk or thickness of the fluidic sealing means 15, the greater will be the positioning distance of the rack 24, 24' with respect to the lying plane P, and therefore with respect to the fluidic sealing means 15.

The plates 26, 26' are therefore substantially parallel to the lateral walls 21, 21' of the rotatable door 13, which is disposed between the plates.

The racks 24, 24' too are substantially parallel to the lateral walls 21, 21' of the rotatable door 13 and hence to the plates 26, 26'.

The plates 26, 26' are attached to a wall 25 of the treatment machine 10, in particular the wall 25 where the access aperture 12 is made.

For example, it can be assumed that the plates 26, 26' are made in a single piece with other plates 22, 22', able to rest and be attached to the wall 25 of the treatment machine 10 by means of attachment elements 20, 20', such as pins, bolts or suchlike.

From the previous description, the functioning of the hinging device 19, 19' of the present treatment machine 10 has certainly already been understood.

In any case, let us suppose that, following a certain operation or treatment cycle, the treatment chamber 11 is closed by the rotatable door 13 as in the situation in fig. 3.

If it becomes necessary to open the rotatable door 13, for example at the end of the operation or treatment cycle, or for any other reason, the drive means 18 are driven so as to rotate the rotatable door 13 from the position in fig. 3 to the position in fig. 5, therefore a completely open position.

It should be noted that the rotatable door 13 could also be opened or closed manually, therefore without the aid of drive means.

The rotatable door 13 substantially rotates around its own hinging axis A, however, advantageously, the hinging axis A, during the rotation of the rotatable door 13, for example on opening, does not remain fixed in position as happens in normal rotatable doors, but moves with respect to the lying plane P, in this case moving away from it.

The displacement of the hinging axis A away from the closed position shown in fig. 3 to the open position shown in fig. 5 allows to gradually and suitably move the corner 17 of the rotatable door 13 away from the fluidic sealing means 15, so that the corner 17 does not interfere with the fluidic sealing means 15.

As can be seen in the intermediate step shown in fig. 4, during the opening rotation R1 of the rotatable door 13, the corner 17 of the rotatable door 13 is already moving away from the fluidic sealing means 15.

As previously mentioned, when opening is complete, the hinging axis A will be at a distance DA greater than the distance DC which it had when the rotatable door 13 was closed.

The distancing movement of the opening hinging axis A is advantageously allowed, in a simple and effective manner, by the roto-translation of the toothed wheels 23, 23', associated with the rotatable door 13, on the corresponding racks 24, 24'.

Naturally, during a closing rotation R2 of the rotatable door 13, for example to move from the situation in fig. 1 or fig. 5 to the situation in fig. 3, the roto-translation of the toothed wheels 23, 23' on the racks 24, 24' causes the hinging axis A to move closer again to the lying plane P and also causes an adequate compression of the fluidic sealing means 15.

By means of the present hinging device 19, 19', therefore, in the case of fluidic sealing means 15 of the inflatable type, the operation to deflate or dilate the fluidic sealing means 15 could also be advantageously rendered superfluous, when it is desired to open the rotatable door 13.

In the examples shown it has been assumed that the movement of the hinging axis A toward or away from the lying plane P occurs linearly, therefore by means of a two-directional roto-translation, but, naturally, an arcuate movement could also be provided, for example by providing grooves 27, 27' and racks 24, 24' having a certain curvature.

To allow sufficient stability and alignment in the attachment of the racks 24, 24', two attachment elements 28, 28' are also shown by way of example, inserted in corresponding slits 29, 29' but, naturally, the number and disposition of the attachment elements 28, 28' and the slits 29, 29' could be different from what is shown here. In particular, it is possible to provide at least one pair of slits 29, 29' aligned in the direction of two-directional translation of the hinging axis A, so as to guarantee greater efficiency, stability and precision of adjustment.

In the case shown here by way of example, the attachment elements 28, 28' and the slits 29, 29' are aligned along the direction of two-directional translation of the hinging axis A, hence in a direction parallel to direction O.

In the present treatment machine 10, therefore, one or more fluidic sealing means 15 can be applied, of the traditional or pneumatic type, in an effective and precise manner and without the risk that, in determinate movement steps of the rotatable door 13, in particular during opening, the rotatable door 13 could interfere with the fluidic sealing means 15, so as to prevent them from being undesirably compressed or crushed in any way, causing a potential deterioration thereof.

It is also obvious that the present hinging device could be advantageously applied also to a treatment machine provided with a rotatable door of a different type from the one shown here, for example a rotatable swing door with a vertical hinging axis.

It is clear that modifications and/or additions of parts can be made to the treatment machine as described heretofore, without departing from the field and scope of the present invention.

It is also clear that, although the present invention has been described with reference to some specific examples, a person of skill in the art shall certainly be able to achieve many other equivalent forms of treatment machine, having the characteristics as set forth in the claims and hence all coming within the field of protection defined thereby.

In the following claims, the sole purpose of the references in brackets is to facilitate reading: they must not be considered as restrictive factors with regard to the field of protection claimed in the specific claims.

## Claims

1. Machine for treating objects, in particular for washing, thermal disinfection and/or sterilization of objects, comprising a treatment chamber (11) provided with an access aperture (12) defining a corresponding lying plane (P); a rotatable door (13) configured to rotate with respect to a corresponding hinging axis (A) in order to open and close said access aperture (12); and fluidic sealing means (15) positioned between said access aperture (12) and said rotatable door (13);
wherein said rotatable door (13) is associated with said access aperture (12) by a hinging device (19, 19') provided with a movement unit (23, 23', 24, 24') to move the hinging axis (A) of the rotatable door (13) with respect to said lying plane (P), so that said hinging axis (A) can be distanced from said lying plane (P) following an opening rotation (R1) of the rotatable door (13) and can move closer to said lying plane (P) following a closing rotation (R2) of said rotatable door (13), wherein the machine comprises at least one adjustment device (28, 28', 29, 29') configured to adjust the position of said movement unit (23, 23', 24, 24') with respect to said lying plane (P) and said fluidic sealing means (15), wherein said movement unit (23, 23', 24, 24') comprises at least a first pair of gears (23, 23') associated with said rotatable door (13) and able to engage with a second pair of gears (24, 24') associated with a wall (25) of the treatment chamber (11); said treatment machine being **characterized in that** said movement unit (23, 23', 24, 24') is an actuator to convert the rotary motion of the rotatable door (13) into a two-directional linear translation motion of said hinging axis (A), following the rotation of the rotatable door (13), so that the hinging axis (A) is at a minimum distance (DC) from said lying plane (P) when said rotatable door (13) is closed, and at a maximum distance (DA) from said lying plane (P) when said rotatable door (13) is completely open and **in that** said adjustment device (28, 28', 29, 29') comprises attachment elements (28, 28') inserted into suitable slits (29, 29') made on said second pair of gears (24, 24') associated with a wall (25) of the treatment chamber (11).

2. Treatment machine as in claim 1, **characterized in that** said attachment elements (28, 28') and said slits (29, 29') are aligned in the direction of two-directional translation of the hinging axis (A).

3. Treatment machine as in claim 1, **characterized in that** it comprises a pair of slits (29, 29') aligned in said direction of two-directional translation of the hinging axis (A).

4. Treatment machine as in claim 1, **characterized in that** said gears (23, 23', 24, 24') comprise at least a first pair of toothed wheels (23, 23') positioned on said hinging axis (A) and able to engage in corresponding racks (24, 24') associated with said wall (25) of the treatment chamber (11).

5. Treatment machine as in claim 4, **characterized in that** said toothed wheels (23, 23') are each associated with a lateral wall (21, 21') of the rotatable door (13) and in proximity to a lower side (16') of the rotatable door (13), said lower side (16') being positioned in proximity to said hinging axis (A) of the rotatable door (13).

6. Treatment machine as in claim 5, **characterized in that** each of said toothed wheels (23, 23') is able to protrude from a guide groove (27, 27') made on a plate (26, 26') associated with said wall (25) of the treatment chamber (11).

7. Treatment machine as in claim 6, **characterized in that** said racks (24, 24') are associated, by means of suitable attachment elements (28, 28') inserted in said slits (29, 29'), with said plate (26, 26') associated with said wall (25) of the treatment chamber (11).

## Patentansprüche

1. Maschine zum Behandeln von Gegenständen, insbesondere zum Waschen, thermischen Desinfizieren und/oder Sterilisieren von Gegenständen, umfassend eine Behandlungskammer (11) mit einer Zugangsöffnung (12), die eine entsprechende Liegeebene (P) definiert; eine drehbare Tür (13), die dazu konfiguriert ist, sich in Bezug auf eine entsprechende Gelenkachse (A) zu drehen, um die besagte Zugangsöffnung (12) zu öffnen und zu schließen; ein fluidisches Dichtmittel (15), das zwischen der Zugangsöffnung (12) und der drehbaren Tür (13) angeordnet ist, wobei die drehbare Tür (13) mit der Zugangsöffnung (12) durch eine Gelenkvorrichtung (19, 19') verbunden ist, die eine Bewegungseinheit (23, 23', 24, 24') aufweist, um die Gelenkachse (A) der drehbaren Tür (13) im Verhältnis zur Liegeebene (P) zu bewegen, so dass sich die Gelenkachse (A) infolge einer Öffnungsdrehung (R1) der drehbaren Tür (13) von der Liegeebene (P) entfernt und infolge einer Schließdrehung (R2) der drehbaren Tür (13) zu der Liegeebene (P) hinbewegt, wobei die Maschine wenigstens eine Einstellvorrichtung (28, 28', 29, 29') umfasst, die dazu konfiguriert ist, die Position der Bewegungseinheit (23, 23', 24, 24') im Verhältnis zu der Liegeebene (P) und dem fluidischen Dichtmittel (15) einzustellen, wobei die Bewegungseinheit (23, 23', 24, 24') wenigstens ein erstes Getriebepaar (23, 23') umfasst, das an der drehbaren Tür (13) angeordnet ist und in der Lage ist, in ein zweites Getriebepaar (24, 24') einzugreifen, das an einer Wand (25) der Behandlungskammer (11) angeordnet ist; wobei die Behandlungsmaschine
**dadurch gekennzeichnet ist, dass** die Bewegungseinheit (23, 23', 24, 24') ein Aktuator ist, um die Drehbewegung der drehbaren Tür (13) in eine zweidirektionale lineare Verschiebung der Gelenkachse (A) umzuwandeln, die der Drehbewegung der drehbaren Tür (13) folgt, so dass die Gelenkachse (A) einen Mindestabstand (DC) von der Liegeebene (P) aufweist, wenn die drehbare Tür (13) geschlossen ist, und einen Maximalabstand (DA) von der Liegeebene (P) aufweist, wenn die drehbare Tür (13) vollständig geöffnet ist, und dass die Einstellvorrichtung (28, 28', 29, 29') Befestigungselemente (28, 28') umfasst, die in geeignete Schlitze (29, 29') eingesteckt sind, die in dem zweiten Getriebepaar (24, 24') ausgebildet sind, das mit der Wand (25) der Behandlungskammer (11) verbunden ist.

2. Behandlungsmaschine nach Anspruch 1
**dadurch gekennzeichnet, dass**
die Befestigungselemente (28, 28') und Schlitze (29, 29') sich in Richtung der zweidirektionalen Verschiebung der Gelenkachse (A) erstrecken.

3. Behandlungsmaschine nach Anspruch 1
**dadurch gekennzeichnet, dass**
die Maschine ein Paar Schlitze (29, 29') umfasst, das sich in Richtung der zweidirektionalen Verschiebung der Gelenkachse (A) erstreckt.

4. Behandlungsmaschine nach Anspruch 1
**dadurch gekennzeichnet, dass**
die Getriebe (23, 23', 24, 24') wenigstens ein erstes Zahnradpaar (23, 23') umfassen, die an der Gelenkachse (A) angeordnet sind, und in der Lage sind mit entsprechenden Zahnstangen (24, 24'), die an der Wand (25) der Behandlungskammer (11) angeordnet sind, einzugreifen.

5. Behandlungsmaschine nach Anspruch 4
**dadurch gekennzeichnet, dass**
die Zahnräder (23, 23') jeweils an einer Seitenwand (21, 21') und in der Nähe einer Unterseite (16') der drehbaren Tür (13) angeordnet sind, wobei die Unterseite (16') in der Nähe zu der Gelenkachse (A) der drehbaren Tür (13) angeordnet ist.

6. Behandlungsmaschine nach Anspruch 5
**dadurch gekennzeichnet, dass**
jedes der Zahnräder (23, 23') dazu ausgebildet ist, aus einer Führungsnut (27, 27') vorzustehen, die auf einer Platte (26, 26') ausgebildet ist, die mit der Wand (25) der Behandlungskammer (11) verbunden ist.

7. Behandlungsmaschine nach Anspruch 6
**dadurch gekennzeichnet, dass**
die Zahnstangen (24, 24') mittels geeigneter Befestigungselemente (28, 28'), die in die Schlitze (29, 29') eingesetzt sind, mit der Platte (26, 26') verbunden sind, die mit der Wand (25) der Behandlungskammer (11) verbunden ist.

## Revendications

1. Machine de traitement d'objets, notamment de lavage, de désinfection thermique et/ou de stérilisation d'objets, comprenant une chambre de traitement (11) munie d'une ouverture d'accès (12) définissant un plan de couchage correspondant (P) ; une porte rotative (13) configurée pour tourner par rapport à un axe d'articulation correspondant (A) afin d'ouvrir et de fermer ladite ouverture d'accès (12) ; et un moyen d'étanchéité fluidique (15) positionné entre ladite ouverture d'accès (12) et ladite porte rotative (13) ; dans laquelle ladite porte rotative (13) est associée à ladite ouverture d'accès (12) par un dispositif d'articulation (19, 19') pourvu d'une unité de déplacement (23, 23', 24, 24') pour déplacer l'axe d'articulation (A) de la porte rotative (13) par rapport audit plan de couchage (P), de sorte que ledit axe d'articulation (A) peut être éloigné dudit plan de couchage (P) suite à une rotation d'ouverture (R1) de la porte rotative (13) et peut se rapprocher dudit plan de couchage (P) suite à une rotation de fermeture (R2) de ladite porte rotative (13), dans laquelle la machine comprend au moins un dispositif d'ajustement (28, 28', 29, 29') configuré pour ajuster la position de ladite unité de déplacement (23, 23', 24, 24') par rapport audit plan de couchage (P) et audit moyen d'étanchéité fluidique (15), dans laquelle ladite unité de déplacement (23, 23', 24, 24') comprend en au moins une première paire d'engrenages (23, 23') associée à ladite porte rotative (13) et apte à venir en prise avec une seconde paire d'engrenages (24, 24') associée à une paroi (25) de la chambre de traitement (11); ladite machine de traitement étant **caractérisée en ce que** ladite unité de déplacement (23, 23', 24, 24') est un actionneur pour convertir le mouvement de rotation de la porte rotative (13) en un mouvement de translation linéaire bidirectionnelle dudit axe d'articulation (A), suite à la rotation de la porte rotative (13), de sorte que l'axe d'articulation (A) est à une distance minimum (DC) dudit plan de couchage (P) lorsque ladite porte rotative (13) est fermée, et à une distance maximum (DA) dudit plan de couchage (P) lorsque ladite porte rotative (13) est complètement ouverte, et **en ce que** ledit dispositif d'ajustement (28, 28', 29, 29') comprend des éléments de fixation (28, 28') insérés dans des fentes appropriées (29, 29') réalisées sur ladite seconde paire d'engrenages (24, 24') associées à une paroi (25) de la chambre de traitement (11).

2. Machine de traitement selon la revendication 1, **caractérisée en ce que** lesdits éléments de fixation (28, 28') et lesdites fentes (29, 29') sont alignés dans le sens de translation bidirectionnelle de l'axe d'articulation (A).

3. Machine de traitement selon la revendication 1, **caractérisée en ce qu'**elle comprend une paire de fentes (29, 29') alignées dans ladite direction de translation bidirectionnelle de l'axe d'articulation (A).

4. Machine de traitement selon la revendication 1, **caractérisée en ce que** lesdits engrenages (23, 23', 24, 24') comprennent au moins une première paire de roues dentées (23, 23') positionnées sur ledit axe d'articulation (A) et aptes à venir dans des crémaillères correspondantes (24, 24') associées à ladite paroi (25) de la chambre de traitement (11).

5. Machine de traitement selon la revendication 4, **caractérisée en ce que** lesdites roues dentées (23, 23') sont associées chacune à une paroi latérale (21, 21') de la porte rotative (13) et à proximité d'un côté inférieur (16') de la porte rotative (13), ledit côté inférieur (16') étant positionné à proximité dudit axe d'articulation (A) de la porte rotative (13).

6. Machine de traitement selon la revendication 5, **caractérisée en ce que** chacune desdites roues dentées (23, 23') est apte à faire saillie à partir d'une rainure de guidage (27, 27') réalisée sur une plaque (26, 26') associée à ladite paroi (25) de la chambre de traitement (11).

7. Machine de traitement selon la revendication 6, **caractérisée en ce que** lesdits crémaillères (24, 24') sont associées, au moyen d'éléments de fixation appropriés (28, 28') insérés dans lesdites fentes (29, 29'), à ladite plaque (26, 26') associée à ladite paroi (25) de la chambre de traitement (11).
